# EUROPEAN PATENT APPLICATION

(11) **EP 2 630 960 A1**
(43) Date of publication of application: **28.08.2013**
(21) Application number: 13167916.9
(22) Date of filing: 19.04.2005
(51) Int. Cl.: A61K 35/62, A61K 9/00, A61K 47/02, A61K 47/18

(54) **Transdermal Delivery of Beneficial Substances Effected by a Hostile Biophysical Environment**

(30) Priority: 19.04.2004 US 563551 P; 19.04.2004 US 563552 P; 19.04.2004 US 563553 P; 19.04.2004 US 563554 P; 19.04.2004 US 563555 P; 19.04.2004 US 563556 P; 19.04.2004 US 563557 P; 19.04.2004 US 563558 P; 19.04.2004 US 563559 P; 19.04.2004 US 563560 P; 19.04.2004 US 563561 P; 19.04.2004 US 563562 P; 19.04.2004 US 563563 P; 19.04.2004 US 563564 P; 19.04.2004 US 563565 P; 19.04.2004 US 563566 P; 19.04.2004 US 563567 P; 19.04.2004 US 563569 P; 19.04.2004 US 563570 P; 19.04.2004 US 563571 P; 19.04.2004 US 563572 P; 19.04.2004 US 563573 P; 19.04.2004 US 563574 P; 19.04.2004 US 563575 P; 19.04.2004 US 563576 P
(62) Divisional of application: 05737763.2
(71) Applicant: Strategic Science & Technologies, LLC, Cambridge MA 02141 (US)
(72) Inventor: Fossel, Eric Thor, Cambridge, MA Massachusetts 02139 (US)
(74) Representative: Heare, Tanya

(57) **Abstract**

The present invention generally relates to the transdermal delivery of substances and, in some embodiments, to the transdermal delivery of beneficial substances by a hostile biophysical environment. In one aspect, various methods for the transdermal delivery of beneficial substances are disclosed. By creating a hostile biophysical environment, beneficial substances may be delivered, according to certain embodiments, through the stratum corneum of the skin into the body. Beneficial substances include, but are not limited to, pharmaceutical agents, drugs, vitamins, co-factors, peptides, dietary supplements, and others. The beneficial effects disclosed include, for instance, relief of pain and inflammation, prevention and healing of ulcers of the skin, relief of headache, improved sexual function and enjoyment, growth of hair on the scalp, improving muscle size and/or function, removing body fat and/or cellulite, treating cancer, treating viral infections and others. A hostile biophysical environment may also be used in conjunction with systems and methods for increasing local blood flow, according to one set of embodiments. For example, by using a nitric oxide donor such as L-arginine, local blood flow may be increased, e.g., by transdermally delivering the nitric oxide precursor. The nitric oxide donor may be the sole cause of increased blood flow, or it may be supplemented with an adjunct such as theophylline.

## Description

### RELATED APPLICATIONS

This application claims the benefit of U.S. Provisional Patent Application Serial No. 60/563,573, filed April 19, 2004, entitled "Flow Assisted Transdermal Preparations of Ephedra and Ephedra Components to Avoid Adverse Effects," by E.T. Fossel; U.S. Provisional Patent Application Serial No. 60/563,575, filed April 19, 2004, entitled "Flow Assisted Transdermal Delivery of Anabolic Steroids," by E.T. Fossel; U.S. Provisional Patent Application Serial No. 60/563,574, filed April 19, 2004, entitled "Augmented Flow Assisted Transdermal Delivery of Anabolic Steroids," by E.T. Fossel; U.S. Provisional Patent Application Serial No. 60/563,558, filed April 19, 2004, entitled "Flow Assisted Topical Transdermal Methods of Drug Delivery," by E.T. Fossel; U.S. Provisional Patent Application Serial No. 60/563,559, filed April 19, 2004, entitled "Transdermal Delivery of Pharmaceutical Agents Effected by a Hostile Biophysical Environment," by E.T. Fossel; U.S. Provisional Patent Application Serial No. 60/563,560, filed April 19, 2004, entitled "Transdermal Drug Delivery by Means of a High Ionic Strength Environment," by E.T. Fossel; U.S. Provisional Patent Application Serial No. 60/563,576, filed April 19, 2004, entitled "Transdermal Delivery of Ephedra and Ephedra Components by Use of A Hostile Biophysical Environment," by E.T. Fossel; U.S. Provisional Patent Application Serial No. 60/563,572, filed April 19, 2004, entitled "An Augmented Flow Assisted Preparation to Increase Muscle Size and Performance," by E.T. Fossel; U.S. Provisional Patent Application Serial No. 60/563,564, filed April 19, 2004, entitled "A Flow Assisted Preparation to Increase Muscle Size and Performance," by E.T. Fossel; U.S. Provisional Patent Application Serial No. 60/563,570, filed April 19, 2004, entitled "Transdermal Preparations to Improve Muscle Function and Size," by E.T. Fossel; U.S. Provisional Patent Application Serial No. 60/563,561, filed April 19, 2004, entitled "Use of a Silicon Based Matrix for Transdermal Delivery of L-Arginine and Adjuncts to Cause Beneficial Effects," by E.T. Fossel; U.S. Provisional Patent Application Serial No. 60/563,562, filed April 19, 2004, entitled "Transdermal Preparation of Ibuprofen to Reduce Pain and Inflammation," by E.T. Fossel; U.S. Provisional Patent Application Serial No. 60/563,567, filed April 19, 2004, entitled "A Transdermal Augmented L-Arginine Preparation for Treatment of Headache," by E.T. Fossel; U.S. Provisional Patent Application Serial No. 60/563,552, filed April 19, 2004, entitled "Flow Assisted Topical Transdermal Method of Drug Delivery of Drugs with Systemic Toxicity," by E.T. Fossel; U.S. Provisional Patent Application Serial No. 60/563,569, filed April 19, 2004, entitled "Transdermal Delivery of Systematically Toxic Pharmaceutical Agents Effected by a Hostile Biophysical Environment," by E.T. Fossel; and U.S. Provisional Patent Application Serial No. 60/563,571, filed April 19, 2004, entitled "Transdermal Flow Assisted Localized Delivery of Chemotherapeutic Agents," by E.T. Fossel.

This application also claims the benefit of U.S. Provisional Patent Application Serial No. 60/563,563, filed April 19, 2004, entitled "Use of Transdermal L-Arginine and Adjuncts to Cause Beneficial Effects by Increasing Local Blood Flow," by E.T. Fossel; U.S. Provisional Patent Application Serial No. 60/563,565, filed April 19, 2004, entitled "Use of Arginine and Arginine Derivatives and Adjuncts to Improve Grafting of Real and Artificial Skin," by E.T. Fossel; U.S. Provisional Patent Application Serial No. 60/563,566, filed April 19, 2004, entitled "Transdermal Delivery of L-Arginine for the Purpose of Enhancing the Appearance of the Female Breast," by E.T. Fossel; U.S. Provisional Patent Application Serial No. 60/563,553, filed April 19, 2004, entitled "Use of A Transdermal L-Arginine and Adjuncts to Improve Bone Healing," by E.T. Fossel; U.S. Provisional Patent Application Serial No. 60/563,554, filed April 19, 2004, entitled "Use of a Transdermal Preparation of L-Arginine and Adjuncts to Effect Wound Healing," by E.T. Fossel; U.S. Provisional Patent Application Serial No. 60/563,555, filed April 19, 2004, entitled "Use of a Transdermal Preparation of L-Arginine and Adjuncts to Facilitate Healing of Infection," by E.T. Fossel; U.S. Provisional Patent Application Serial No. 60/563,556, filed April 19, 2004, entitled "Transdermal Delivery of L-Arginine Preparation to Regress Neuropathy and Heal and Prevent Ulcers," by E.T. Fossel; U.S. Provisional Patent Application Serial No. 60/563,557, filed April 19, 2004, entitled "A Transdermal Preparation of L-Arginine to Improve Flow in Peripheral Artery Disease and Prevent Claudication," by E.T. Fossel; and U.S. Provisional Patent Application Serial No. 60/563,551, filed April 19, 2004, entitled "Use of a Transdermal L-Arginine Preparation and Adjuncts to Improve Outcome in Transplant and Plastic Surgery," by E.T. Fossel.

Each of the above applications is incorporated herein by reference.

### FIELD OF INVENTION

The present invention generally relates to the transdermal delivery of substances.

### BACKGROUND

Local transdermal delivery of drugs, while desirable, is limited by current technologies. Few pharmaceutical entities have successfully been delivered transdermally in effective dosages. For example, a limited number of drugs, such as steroids, nicotine and nitroglycerine, which are non-charged and do not form hydrogen bonds, have been successfully delivered by passive diffusion, relying on the concentration gradient between outside and inside the skin to deliver the agent in accordance with Fick's first law of diffusion. The amount of pharmaceutical agent that can be delivered through simple diffusion is also limited. For instance, once the concentration inside the stratum corneum becomes equal to that outside, flow of pharmaceutical agent may stop. Thus, improvements in the transdermal delivery of substances, locally or systemically, are needed.

### SUMMARY OF THE INVENTION

The present invention generally relates to the transdermal delivery of substances, locally or systemically, and in some embodiments, to the transdermal delivery of beneficial substances by a hostile biophysical environment. The subject matter of the present invention involves, in some cases, interrelated products, alternative solutions to a particular problem, and/or a plurality of different uses of one or more systems and/or articles.

This invention relates, in one aspect, to the field of localized transdermal delivery of substances which have a beneficial effect, for example, to the transdermal delivery of herbs, vitamins, minerals, pharmaceutical agents, drugs, peptides, dietary supplements, or other substances affected by a hostile biophysical environment. The hostile biophysical environment may comprise a high ionic strength vehicle in some embodiments, and delivery may be enhanced, in certain cases, by various techniques to increase local blood flow at the delivery site. For instance, substances with localized action may avoid systemic toxicity if delivered locally and transdermally. In some cases, various beneficial substances which function at local sites, rather than systemically, may be more efficaciously administered by transdermal rather than systemic administration. If the beneficial substance is delivered through the skin, a higher dose in the tissue to be treated may be achieved. In addition, a substantially lower total body dose may be achieved in some cases. This can be understood if one considers the non-limiting example of treating pain in finger joints with a NSAID (a nonsteroidal anti-inflammatory drug). If the pain is to be treated systemically, e.g., by oral administration, the whole body, including the finger joints, is dosed with the NSAID. The concentration of NSAID is approximately the same throughout the body, including the finger joints. If, on the other hand, one were to apply the NSAID transdermally to the finger joints, the rest of the body would not be dosed (or would be dosed to a significantly lesser extent). Thus, the total dose of transdermal NSAID would only be a fraction of the dosage required for systemic administration.

In some embodiments, the beneficial substances delivered transdermally may improve health, improve body function, or treat a variety of disease states.

In one set of embodiments, the invention relates to the field of headache treatment, and in some cases, to the use of arginine and/or arginine derivatives or adjuncts to provide effective headache relief. This invention also relates, in another set of embodiments, to the field of relief of pain and/or inflammation and in some cases, to a transdermal preparation of ibuprofen to reduce pain and/or inflammation. In certain instances, the ibuprofen is delivered from a vehicle into the tissue through the use of a hostile biophysical environment.

This invention also relates, in yet another set of embodiments, to systems and methods for improving uptake of muscle improving agents, for example, by increasing local blood flow by delivering a nitric oxide donor such as L-arginine, either alone or with an adjunct such as theophylline. This invention also relates, in still another set of embodiments, to topical methods of administrating anabolic steroids, for example, steroids that exhibit unacceptable systemic toxicity. The steroids may also promote improved muscle size and function through the use of enhanced blood flow.

This invention relates, in one set of embodiments, to topical methods of administering chemotherapeutic or antiviral agents, for instance, to promote healing or recovery, or to prevent recurrence of a localized cancer or viral infection.

In yet another set of embodiments, this invention relates to the field of enhanced sexual function. In some cases, arginine and/or arginine derivatives and adjuncts may be applied to increase genital blood flow, which may increase sexual function.

In one aspect, the invention is a method. The method includes, in one set of embodiments, an act of applying, to a portion of the skin of a subject, a delivery vehicle comprising a pharmaceutical agent in a hostile biophysical environment.

In another aspect, the invention includes a delivery vehicle. In one set of embodiments, the delivery vehicle includes a nitric oxide donor, and a pharmaceutical agent at a dosage effective to treat a localized medical condition, wherein the dosage is lower than the effective dosage of the pharmaceutical agent when taken orally. In another set of embodiments, the delivery vehicle includes a nitric oxide donor, and a pharmaceutical agent able to treat one or more medical conditions selected from the group consisting of cramps, pain, migraine, arthritis, swelling, sexual dysfunction, hair loss, skin ulcers, and migraine.

One aspect of the invention is directed to a method comprising an act of administering, to a subject, a delivery vehicle comprising a nitric oxide donor contained within a hostile biophysical environment. Another aspect of the invention is directed to an article comprising a cream containing a nitric oxide donor in a hostile biophysical environment.

Several methods are disclosed herein of administering a subject (which may be human or a non-human animal) with a composition for prevention or treatment of a particular condition. It is to be understood that in each such aspect of the invention, the invention specifically includes, also, the composition for use in the treatment or prevention of that particular condition, as well as use of the composition for the manufacture of a medicament for the treatment or prevention of that particular condition.

The present invention, in another aspect, is directed to a method of making one or more of the embodiments described herein. In yet another aspect, the present invention is directed to a method of using one or more of the embodiments described herein. In still another aspect, the present invention is directed to a method of promoting one or more of the embodiments described herein.

Other advantages and novel features of the present invention will become apparent from the following detailed description of various non-limiting embodiments of the invention. In cases where the present specification and a document incorporated by reference include conflicting and/or inconsistent disclosure, the present specification shall control. If two or more documents incorporated by reference include conflicting and/or inconsistent disclosure with respect to each other, then the document having the later effective date shall control.

### DETAILED DESCRIPTION

The present invention generally relates to the transdermal delivery of substances and, in some embodiments, to the transdermal delivery of beneficial substances by a hostile biophysical environment. In one aspect, various methods for the transdermal delivery of beneficial substances are disclosed. By creating a hostile biophysical environment, beneficial substances may be delivered, according to certain embodiments, through the stratum corneum of the skin into the body. Beneficial substances include, but are not limited to, pharmaceutical agents, drugs, vitamins, co-factors, peptides, dietary supplements, and others. The beneficial effects disclosed include, for instance, relief of pain and inflammation, prevention and healing of ulcers of the skin, relief of headache, improved sexual function and enjoyment, growth of hair on the scalp, improving muscle size and/or function, removing body fat and/or cellulite, treating cancer, treating viral infections and others. A hostile biophysical environment may also be used in conjunction with systems and methods for increasing local blood flow, according to one set of embodiments. For example, by using a nitric oxide donor such as L-arginine, local blood flow may be increased, e.g., by transdermally delivering the nitric oxide precursor. The nitric oxide donor may be the sole cause of increased blood flow, or it may be supplemented with an adjunct such as theophylline.

The following documents are incorporated herein by reference: U.S. Provisional Patent Application Serial Nos. 60/563,563, 60/563,558, 60/563,559, 60/563,560, 60/563,561, 60/563,562, 60/563,572, 60/563,564, 60/563,565, 60/563,566, 60/563,567, 60/563,553, 60/563,554, 60/563,555, 60/563,556, 60/563,557, 60/563,551, 60/563,552, 60/563,569, 60/563,570, 60/563,571, 60/563,573, 60/563,574, 60/563,575, and 60/563,576, each filed April 19, 2004, by E.T. Fossel; U.S. Provisional Patent Application Serial No. 60/546,214, filed February 23, 2004, entitled "Topical Delivery of a Nitric Oxide Donor to Improve Body and Skin Appearance," by E.T. Fossel; U.S. Patent Application Serial No. 08/932,227, filed September 17, 1997, entitled "Topical Delivery of Arginine of Cause Beneficial Effects," by E.T. Fossel, published as 2002/0041903 on April 11, 2002; U.S. Patent Application Serial No. 10/201,635, filed July 22, 2002, entitled "Topical Delivery of L-Arginine to Cause Beneficial Effects," by E.T. Fossel, published as 2003/0028169 on February 6, 2003; U.S. Patent Application Serial No. 10/213,286, filed August 5, 2002, entitled "Topical and Oral Arginine to Cause Beneficial Effects," by E.T. Fossel, published as 2003/0018076 on January 23, 2003; International Patent Application No. PCT/US98/19429, filed September 17, 1998, entitled "A Delivery of Arginine to Cause Beneficial Effects," by E.T. Fossel, published as WO 99/13717 on March 25, 1999; U.S. Patent No. 5,895,658, issued April 20, 1999, entitled "Topical Delivery of L-Arginine to Cause Tissue Warming," by E.T. Fossel; U.S. Patent No. 5,922,332, issued July 13, 1999, entitled "Topical Delivery of Arginine to Overcome Pain," by E.T. Fossel; U.S. Patent No. 6,207,713, issued March 27,2001, entitled "Topical and Oral Delivery of Arginine to Cause Beneficial Effects," by E.T. Fossel; U.S. Patent No. 6,458,841, issued October 1, 2002, entitled "Topical and Oral Delivery of Arginine to Cause Beneficial Effects," by E.T. Fossel; International Patent Application No. PCT/US2005/005726, filed February 23, 2005, entitled "Topical Delivery of a Nitric Oxide Donor to Improve Body and Skin Appearance," by E.T. Fossel; and an international patent application filed on even date herewith, entitled "Use of Transdermal L-Arginine and Adjuncts to Cause Beneficial Effects by Increasing Local Blood Flow," by E.T. Fossel.

Detailed descriptions of the various embodiments are provided herein. It is to be understood, however, that the present invention may be embodied in various forms. Therefore, specific details disclosed herein are not to be interpreted as limiting, but rather as a basis for the claims and as a representative basis for teaching one skilled in the art to employ the present invention in virtually any appropriately detailed system, structure, or manner.

One aspect of the invention provides for the delivery of beneficial substances such as pharmaceutical agents (e.g., drugs, biological compounds, etc.) into the body, and such treatments may be systemic or localized, e.g., directed to a specific location of the body, such as the head, one or more specific muscles, the genitals, etc., depending on the specific application.

In one set of embodiments, pharmaceutical agents are introduced to aid in treatment of medical conditions or diseases, and the symptoms associated thereof. In some embodiments, the invention provides for the treatment of medical conditions or diseases and/or ailments using pharmaceutical agents (for example, to treat a subject diagnosed with a medical condition or disease, as described herein), and in some cases, the invention provides for the delivery of a minimum amount of pharmaceutical agents to provide effective levels of medication to an effected area topically while limiting side effects. In some cases, the effective dosage of the pharmaceutical agent may be lower than the effective dosage of the pharmaceutical agent when taken orally. Other embodiments of the invention provides methods for treating cancer, viral infections, erectile dysfunction, sexual dysfunction, an ulcer, swelling, or arthritis. Still another embodiment of the invention provides methods for treating pain, for example, pain from migraine, other headaches, joint pain, muscle pain and other types of pain, Yet another embodiment of the present invention provides methods for restoring hair growth, for example, on a portion of the scalp, which may be scarce in hair.

Non-limiting examples of pharmaceutical agents include small molecules (e.g., having a molecular weight of less than about 2,000 Da, less than about 1,500 Da, or less than about 1,000 Da), peptides (e.g., having less than about 10, less than about 15, less than about 20, or less than about 25 amino acids), proteins (typically larger than peptides), hormones, vitamins, nucleic acids, or the like. Additional examples of suitable pharmaceutical agents for use with the present invention include, but are not limited to, NSAIDs (nonsteroidal anti-inflammatory drugs) such as acetylsalicylsalicylic acid, naproxen, celecoxib, refecoxib, etc.; pharmaceutical agents with narcotic action such as morphine, codine, propoxyphene, oxycodone, hydrocodon, or other similar narcotics; pharmaceutical agents for erectile or sexual dysfunction such as yohimbie, alprostadil, sildenafil, cialis, uprima, vardenaifl, or the like; pharmaceutical agents for migraine such as dihydroergotamine and its salts, ergotamine and its salts, surnatripan and its salts, rizatriptan and its salts, zolmitriptan and its salts, etc.; pharmaceutical agents for hair treatment such as finasteride, eflornithine, minoxidil, or the like; or other pharmaceutical agents such as niacin, lidocaine, benzocaine, ibuprofen, etc. Additional examples include muscle improving agents, for example, creatine or creatine precursors (e.g., creatine phosphate), arginine and/or other nitric oxide donors, and/or ATP precursors such as, inosine, adenosine, inosine, adenine, hypoxanthine, ribose, phosphate (e.g., monosodium phosphate), etc., and/or anabolic steroid agents, such as androstene, DHEA, androstenediol, androstenedione, or the like. Another example is ephedra or its components, such as ephedrine and pseudoephedrine. Yet another example are chemotherapeutic agents or agents for treating cancer and/or viral infections, for example, but not limited to tamoxifen (e.g., for breast cancer treatment), *cis*-platin, carboplatin and related molecules, chclophosphamide and related molecules, vinca alkaloids, epipodophyllotoxins including taxol, acyclovir, or the like. For example, the cancer and/or viral infections may be skin cancer, breast cancer, penile cancer, testicular cancer, or other localized cancers, or viral infections, such as herpes.

As a particular, non-limiting example, ibuprofen is an effective agent against pain when orally administered. However, it is irritating to the lining of the stomach, and people with a tendency to develop ulcers or have an irritated upper gastrointestinal track are typically warned to avoid the use of ibuprofen. The present invention thus allows the topical application of ibuprofen to the site of inflammation or pain, while avoiding the rest of the body, especially the stomach.

As another particular, non-limiting example, while growth hormones, steroids, supplements, and other such agents have been administered orally and by injection to improve muscle size and function, these muscle improving agents are often distributed throughout the body, resulting in only a small portion of the agent acting at the muscle area being used and developed. Muscle requires both creatine phosphate (CrP) and adenosine triphosphate (ATP) to function. Often muscle has insufficient amounts of these substances and their precursors to maintain high level function. Administration of these substances and their precursors have been attempted but in low dose that is ineffective and in high dose is both very expensive and produces side effects such as gastrointestinal distress. Use of topical transdermal delivery to the desired muscle or muscles of a muscle improving agent, according to various embodiments, may localize the dose to the desired area, and potentially results in a higher concentration of the agent at the desired area.

A variety of methods for effecting or improving absorption of beneficial substances (including pharmaceutical agents) are also included in various aspects of the invention. In some cases, a hostile biophysical environment may be used. In a hostile biophysical environment, the environment surrounding the beneficial substance may be such that the beneficial substance is a chemically/energetically unfavorable environment, relative to the skin (e.g., the chemical potential and/or the free energy of the beneficial substance within the hostile biophysical environment is significantly greater than the chemical potential and/or the free energy of the beneficial substance within the skin, thus energetically favoring transport into the skin), especially the stratum corneum. The hostile biophysical environment which raises the chemical potential and/or the free energy of the beneficial substance can be comprised of a high ionic strength, a high concentration of osmotic agents such as ureas, sugars, or carbohydrates, a high pH environment (e.g., greater than about 9, greater than about 10, greater than about 11, greater than about 12, or greater than about 13), a low pH environment (less than about 5, less than about 4, less than about 3 or less than about 2), highly hydrophobic components, or highly hydrophilic components or other substances that cause an increase in the chemical potential and/or free energy of the beneficial substance. A hydrophobic component may have an octanol-water partition coefficient of at least about 100, at least about 1000, at least about 10⁴, at least about 10⁵, or more in some cases. Similarly, a hydrophilic component may have an octanol-water partition coefficient of less than about 0.01, less than about 10⁻³, less than about 10⁻⁴, or less than about 10⁻⁵ in some cases.

In some cases, the delivery vehicle defines the biophysical hostile environment. In other cases, the beneficial substance may be packaged in such a way that it is carried into tissue and/or its charge is neutralized by derivitization and/or by forming a neutral salt. Examples of biophysically hostile environments include, but are not limited to, high ionic strength environments (e.g., by the addition of ureas, sugars, carbohydrates, and/or ionic salts such as lithium chloride, sodium chloride, potassium chloride, calcium chloride, magnesium chloride, choline chloride, sodium fluoride, lithium bromide, etc., as well as combinations of these and/or other agents, for instance at high ionic strengths (for example, greater than about 0.25 M, greater than about 1 M, greater than about 2 M, greater than about 3 M, greater than about 5 M, greater than about 10 M, greater than about 15 M, greater than about 20 M, greater than about 25 M, etc., or in some cases, between about 0.25 M and about 15 M, between about 5 M and about 15 M, between about 10 M and about 15 M, etc.); high or low pH environments (e.g., by adding pharmaceutically acceptable acids or bases, for example, such that the pH is between about 3 and about 7, between about 3 and about 6, between about 3 and about 5, between about 7 and about 11, between about 8 and about 11, between about 9 and about 11, etc.); or highly hydrophobic environments (e.g., by decreasing water content and increasing lipid, oil and/or wax content of the environment). Other highly charged molecules such as polylysine, polyglutamine, polyaspartate, etc., or copolymers of such highly charged amino acids may also be used in certain embodiments to create the hostile biophysical environment. Non-limiting examples of packaging which would be carried into tissue includes liposomes or emulsions of collagen, collagen peptides or other components of skin or basement membrane. Non-limiting examples of neutralization of charge include delivery of the beneficial substance in the form or an ester or salt which is electronically neutral. In some embodiments, the hostile biophysical environment may include any two or more of these conditions. For instance, the hostile biophysical environment may include high ionic strength and a high pH or a low pH, a highly hydrophobic environment and a high pH or a low pH, a highly hydrophobic environment that includes liposomes, or the like.

A hostile biophysical environment may also be created in some embodiments by placing a beneficial substance that is relatively highly charged into a hydrophobic, oily environment such as in an oil-based cream or lotion containing little or no water. Absorption may further be aided by combining the use of hostile biophysical environments with the use of penetrating agents, as further described below.

It should be noted that a hostile biophysical environment optimized for one beneficial substance may not necessarily be optimal for another beneficial substance. For example, an optimal hostile biophysical environment for a beneficial substance that is non-charged and does not form hydrogen bonds, in one embodiment of the invention, may not necessarily be optimal for other embodiments of the invention, in which a beneficial substance is charged, and/or in embodiments in which the beneficial substance is able to form hydrogen bonds. Thus, different hostile biophysical environment(s) may be prepared or optimized for different application(s) including different beneficial substance(s) being delivered using the hostile biophysical environment(s).

In certain aspects of the invention, a pharmaceutical agent or other beneficial substance may be combined with a penetrating agent, i.e., an agent that increases transport of the pharmaceutical agent or other beneficial substance into the skin, relative to transport in the absence of the pharmaceutical agent or other beneficial substance. In some embodiments, the penetrating agent may be combined with a hostile biophysical environment. Examples of penetrating agents include oleoresin capsicum or its constituents, or certain molecules containing heterocyclic rings to which are attached hydrocarbon chains.

Non-limiting examples of penetrating agents include, but are not limited to, cationic, anionic, or nonionic surfactants (e.g., sodium dodecyl sulfate, polyoxamers, etc.); fatty acids and alcohols (e.g., ethanol, oleic acid, lauric acid, liposomes, etc.); anticholinergic agents (e.g., benzilonium bromide, oxyphenonium bromide); alkanones (e.g., n-heptane); amides (e.g., urea, N,N-dimethyl-m-toluamide); fatty acid esters (e.g., n-butyrate); organic acids (e.g., citric acid); polyols (e.g., ethylene glycol, glycerol); sulfoxides (e.g., dimethylsulfoxide); terpenes (e.g., cyclohexene); ureas; sugars; carbohydrates or other agents. In one embodiment, the penetrating agent includes a salt, e.g., as previously described.

The present invention, in one aspect, provides various systems and techniques for increasing local blood flow. For example, increased blood flow may be used to introduce pharmaceutical agents (e.g., drugs, biological compounds, etc.) to aid in treatment of medical conditions or diseases and the symptoms associated thereof (for example, to treat a subject diagnosed with a medical condition or disease, as described herein), and/or the increased blood flow may be used to provide effective treatment of medical conditions or diseases and/or ailments with the minimum amount of pharmaceutical agents possible to provide effective levels of medication to an effected area topically while limiting side effects. In one set of embodiments, a nitric oxide donor such as L-arginine and/or L-arginine hydrochloride in an effective concentration may be used to increase localized blood flow, which may enhance delivery of a pharmaceutical agent or other beneficial substance, e.g., to locally afflicted tissue. Nitric oxide may relax the blood vessels, allowing for increased blood flow. In some cases, one or more nitric oxide donors (e.g., 2, 3, 4, 5, 6, 7, 8, 9, 10, etc. nitric oxide donors) may be combined with one or more beneficial substances (e.g., 2, 3, 4, 5, 6, 7, 8, 9, 10, etc. beneficial substances) in a suitable hostile biophysical environment, as described herein.

Besides L-arginine and L-arginine hydrochloride, other non-limiting examples of nitric oxide donors include D,L-arginine, D-arginine, or alkyl (e.g., ethyl, methyl, propyl, isopropyl, butyl, isobutyl, *tert*-butyl, etc.) esters of L-arginine and/or D-arginine (e.g., a methyl ester, an ethyl ester, a propyl ester, a butyl ester, etc.) and/or salts thereof, as well as other derivatives of arginine and other nitric oxide donors. For instance, non-limiting examples of pharmaceutically acceptable salts include hydrochloride, glutamate, butyrate, or glycolate (e.g., resulting in L-arginine glutamate, L-arginine butyrate, L-arginine glycolate, D-arginine hydrochloride, D-arginine glutamate, etc.). Other examples of nitric oxide donors include L-arginine-based compounds such as, but not limited to, L-homoarginine, N-hydroxy-L-arginine, nitrosylated L-arginine, nitrosylated L-arginine, nitrosylated N-hydroxy-L-arginine, nitrosylated N-hydroxy-L-arginine, citrulline, ornithine, linsidomine, nipride, glutamine, etc., and salts thereof (e.g., hydrochloride, glutamate, butyrate, glycolate, etc.). Still other non-limiting examples of nitric oxide donors include S-nitrosothiols, nitrites, 2-hydroxy-2-nitrosohydrazines, or substrates of various forms of nitric oxide synthase. In some cases, the nitric oxide may be a compound that stimulates endogenous production of nitric oxide *in vivo.* Examples of such compounds include, but are not limited to, L-arginine, substrates of various forms of nitric oxide synthase, certain cytokines, adenosine, bradykinin, calreticulin, bisacodyl, phenolphthalein, OH-arginine, or endothelein.

It should be understood that, in any of the embodiments described herein that describe L-arginine, other nitric oxide donors may also be used instead, or in combination with, L-arginine, in other embodiments of the invention.

Without wishing to be bound to any theory, it is generally believed that the flow of the pharmaceutical agent or other beneficial substance across the skin may slow as it builds up within the tissue. Fick's first law of diffusion suggests that when the concentration inside becomes substantially equal to that outside, passive flow stops. The increased local blood flow may prevent or at least decrease the stoppage of the flow of the pharmaceutical agent or other beneficial substance. Thus, when the vehicle containing the pharmaceutical agent or other beneficial substance and a nitric oxide donor, such as L-arginine, is applied to the skin, the pharmaceutical agent or other beneficial substance exits the vehicle into the tissue more readily, as the pharmaceutical agent is dispersed by flow and does not build up in concentration in the tissue. Thus, in certain embodiments, pharmaceutical agents or other beneficial substances may be introduced into the skin, for example, ibuprofen, anabolic steroids, or other agents or substances described herein.

One set of embodiments provides for increased blood flow to the genitals, for example, using a nitric oxide donor such as L-arginine, optionally in combination with a silicon-based transdermal preparation and/or an adjunct such as theophylline. Adequate local genital blood flow is important for optimal sexual function and satisfaction in both men and women. In men it is important to achieve and maintain an erection. In women it is important for nerve sensitivity which is required to attain satisfying orgasms. In some cases, the preparation may be contained within a condom, optionally with other sexual-enhancing agents, such as lubricants.

A non-limiting example of such a preparation includes a silicon-based vehicle (e.g., a vehicle that contains a silicon-containing substance) with properties of excellent absorption into the skin which also contains L-arginine hydrochloride (7.5% w/v), theophylline (5% w/v) and a mixture of high molecular weight polydimethylsiloxane and a low viscosity cyclotetrasiloxane (commercially known as Dow Corning 1411 fluid), and water prepared as an emulsion. The silicon emulsion provides a hostile biophysical environment in this example. The emulsion is applied to the genitals (e.g., the penis, or the clitoris and/or the vagina) and rubbed in until absorbed. The emulsion may facilitate enhanced blood flow to the genitals, bringing oxygen and other nutrients and blood to that tissue. In addition, the silicon may act as a lubricant for improved enjoyment of sexual function. Additional preparations are discussed in more detail herein. Other examples of silicon-containing substances include polydimethylsiloxane, cyclopentasiloxane, dimethicol, or dimethicon. For example, a preparation of the invention may be a cream contanining water (20-80%), a polydimethylsiloxane/cyclopentasiloxane mixture (20-90% w/v) and TWEEN 20 (1-10%), and the pH may be between about 3 and about 11.

A "nitric oxide donor," as used herein, is a compound that contains therein a nitric oxide (NO) moiety, where the compound is able to release nitric oxide and/or chemically transfer the nitric oxide moiety to another molecule, directly or indirectly, for example, through a biological process. The nitric oxide donor may release nitric oxide into the skin, and/or tissues such as muscles and/or elements of the circulatory system in close proximity to the surface of the skin. Non-limiting examples of nitric oxide donors include arginine (e.g., L-arginine and/or D-arginine), arginine derivatives (e.g., L-arginine hydrochloride and/or D-arginine hydrochloride), nitroglycerin, polysaccharide-bound nitric oxide-nucleophile adducts, N-nitroso-N-substituted hydroxylamines, 1,3-(nitrooxymethyl)phenyl-2-hydroxybenzoate, etc., as described in more detail herein. In some cases, the concentration of nitric oxide and/or the nitric oxide donor may be tailored to have a duration of effective treatment of at least about 3 hours, at least about 5 hours, or at least about 8 hours or more in certain instances. The duration may also be controlled, for instance, by controlling the concentration of a penetrating agent used in conjunction with nitric oxide and/or the nitric oxide donor. The actual concentration for a particular application can be determined by those of ordinary skill in the art using no more than routine experimentation, for example, by measuring the amount of transport of nitric oxide and/or the nitric oxide donor as a function of concentration *in vitro* across cadaver skin or suitable animal models, skin grafts, synthetic model membranes, or the like.

As a particular non-limiting example, in one embodiment, nitric oxide is provided using L-arginine, for example, at a concentration of at least about 0.5% by weight (wt% or w/v) of L-arginine (optionally with one or more penetrating agents as discussed herein, for example, a penetrating agent able to create a hostile biophysical environment), at least about 0.75 wt%, at least about 1 wt%, at least about 2 wt%, at least about 3 wt%, at least about 5 wt%, at least about 7 wt%, at least about 10 wt%, or at least about 15 wt%. The L-arginine may be present in a suitable delivery vehicle, such as a cream or a lotion. L-arginine may be particularly useful in some cases due to its low toxicity, its high solubility, or its low cost. Other examples of nitric oxide donors are discussed in International Patent Application No. PCT/US2005/005726, filed February 23, 2005, entitled "Topical Delivery of a Nitric Oxide Donor to Improve Body and Skin Appearance," by E.T. Fossel, incorporated herein by reference.

Thus, another aspect of the invention provides for the delivery of nitric oxide and/or nitric oxide donors into the body, as further described below, and such treatments may be systemic or localized, e.g., directed to a specific location of the body, such as the head, arms, legs, feet, etc., depending on the specific application. The nitric oxide and/or nitric oxide donor may increase local blood flow, thereby enhancing tissue health. Increased blood flow may also assist in the healing process, e.g., where injury or surgery has occurred.

In one set of embodiments, nitric oxide and/or a nitric oxide donor (e.g., arginine and/or an arginine derivative), optionally including an adjunct such as theophylline, may be applied to a subject to improve the outcome of various medical conditions, such as surgical treatments (e.g., at a site of surgery). Non limiting for examples include transplant and plastic surgery, graft sites of real or artificial skin, or other surgically treated areas. In some embodiments of the invention, a treatment of the invention may be applied to improve flow in peripheral artery disease and/or prevent claudication, to improve the circulation in the feet of people with diabetes and others with impaired circulation, to regress neuropathy, to heal or prevent ulcers, to improve bone healing, to treat infection (e.g.; bacterial infections, viral infections, fungal infections, etc.), to improve grafting of real or artificial skin, and/or to improve wound healing, and/or to improve a surgically treated area.

In another set of embodiments, nitric oxide and/or a nitric oxide donor (e.g., arginine and/or an arginine derivative), optionally including an adjunct such as theophylline, may be applied to a subject having peripheral artery disease (PAD), for example, in subjects treated invasively or non-invasively. For instance, arteries are often reopened by use of angioplasty, arthectomy or bypass surgery, or through the use of intravenous drug treatments, such as Corlapam, Flolan, or Primacor. Left untreated or unsuccessfully treated, PAD can lead to claudication, which can be incapacitating, resulting not only in great pain but loss of the ability to carry on a normal life. Various systems and methods of the present invention can be used in some cases, as a replacement for and/or in conjunction with such methods of treatment.

Yet another set of embodiments provides for the enhancement of bone healing by increasing local blood flow. Bone healing is a slow and complex process, and it is enhanced by a variety of proteins and cells in the blood. An increase in blood flow rate may thus enhance bone healing. In some cases, the application of a nitric oxide and/or a nitric oxide donor (e.g., arginine and/or an arginine derivative), optionally including an adjunct such as theophylline, may increase blood flow to the bone. Thus, for example, a fractured bone (including a broken bone) may be treated in certain embodiments of the invention.

In still another set of embodiments, an infection may be treated by increasing local blood flow. The body fights infection using cells and cell derived materials found in the blood. Increasing blood flow to the site of an infection can enhance the body's mechanisms for fighting infection. Thus, the application of a nitric oxide and/or a nitric oxide donor (e.g., arginine and/or an arginine derivative), optionally including an adjunct such as theophylline, may increase blood flow to the site of infection, which may promote healing.

Another set of embodiments is generally directed to the treatment of blood flow in persons with diabetes, e.g., in the hands and/or feet. The application of a nitric oxide and/or a nitric oxide donor (e.g., arginine and/or an arginine derivative), optionally including an adjunct such as theophylline, may be used to treat such conditions, thereby increasing blood flow within the hands and/or feet. In some cases, long lasting improvement in blood flow, and/or regression of diabetic neuropathy may be achieved. For example, local blood flow may be increased by at least about 20% or at least about 30%. Still another set of embodiments of the invention are directed to the prevention or treatment of diabetic skin ulcers, e.g., by increasing blood flow, as previously described.

In still another set of embodiments, nitric oxide and/or a nitric oxide donor (e.g., arginine and/or an arginine derivative), optionally including an adjunct such as theophylline, may be applied to the skin, for example, to a skin graft and/or graft material of a skin graft, to a wound in the skin, etc. Often, skin grafts do not have sufficient blood flow, which may lead to graft failure. By enhancing the blood flow to the skin graft, e.g., using the nitric oxide and/or a nitric oxide donor, graft failure may be reduced. In some cases, the nitric oxide and/or nitric oxide donor may be applied to tissues proximate the skin graft, and/or the nitric oxide and/or nitric oxide donor may be induced to migrate to tissues adjacent the skin graft.

Another set of embodiments of the invention is directed to treating medical conditions by not only enhancing blood flow to a treated region of the body, but also by enhancing transdermal delivery of a pharmaceutical agent or other beneficial substance through the use of a nitric oxide and/or a nitric oxide donor (e.g., arginine and/or an arginine derivative), optionally including an adjunct such as theophylline, to increase blood flow at the site of molecular transport. Such embodiments may be relatively simple, inexpensive, and/or non-irritating, and in many cases, no physical or mechanical devices are required. Such transport may be further increased, for example, in combination with penetrating agents or the like, as described herein.

In some aspects of the invention, a nitric oxide and/or nitric oxide donor, and/or a pharmaceutical agent or other beneficial substance, may be administered using a delivery vehicle such as a cream, gel, liquid, lotion, spray, aerosol, or transdermal patch. Examples of delivery vehicles are discussed below. The delivery vehicle may promote transfer into the skin of an effective concentration of the nitric oxide and/or nitric oxide donor, and/or a pharmaceutical agent or other beneficial substance, directly or indirectly. For instance, the delivery vehicle may include one or more penetrating agents, as further described herein. In some embodiments, the delivery vehicle may include a hostile biophysical environment, e.g., using a penetrating agent, etc., as described herein. Those of ordinary skill in the art will know of systems and techniques for incorporating a nitric oxide and/or nitric oxide donor, and/or a pharmaceutical agent or other beneficial substance within delivery vehicles such as a cream, gel, liquid, lotion, spray, aerosol, or transdermal patch. In some cases, the concentration of the nitric oxide and/or nitric oxide donor, and/or a pharmaceutical agent or other beneficial substance in the delivery vehicle can be reduced with the inclusion of a greater amount or concentration of penetrating agent, or increased to lengthen the beneficial effect. In one set of embodiments, the nitric oxide and/or nitric oxide donor, and/or a pharmaceutical agent or other beneficial substance may be used in conjunction with an adjunct, such as theophylline (for example, at 10% weight by volume).

Other materials may be present within the delivery vehicle, for example, buffers, preservatives, surfactants, etc. For instance, the cream may include one or more of water, mineral oil, glyceryl stereate, squalene, propylene glycol stearate, wheat germ oil, glyceryl stearate, isopropyl myristate, steryl stearate, polysorbate 60, propylene glycol, oleic acid, tocopherol acetate, collagen, sorbitan stearate, vitamin A and D, triethanolamine, methylparaben, aloe vera extract, imidazolidinyl urea, propylparaben, PND, or BHA.

As specific non-limiting examples, the cream may have one or more of (w/v): water (20-80%), white oil (3-18%), glyceryl stearate (0.25-12%), squalene (0.25-12%), cetyl alcohol (0.1-11%), propylene glycol stearate (0.1-11%), wheat germ oil (0.1-6%), polysorbate 60 (0.1-5%), propylene glycol (0.05-5%), collagen (0.05-5%), sorbitan stearate (0.05-5%), vitamin A (0.02-4%), vitamin D (0.02-4%), vitamin E (0.02-4%), triethanolamine (0.01-4%), methylparaben (0.01-4%), aloe vera extract (0/01-4%), imidazolidinyl urea (0.01-4%), propylparaben (0.01-4%), BHA (0.01-4%), L-arginine Hydrochloride (0.25-25%), sodium chloride (0.25-25%), magnesium chloride (0.25-25%), and/or choline chloride (0.25-25%). The percentages of each compound can vary (or the compound may be absent in some cases), for example, 1%, 2%, 3%, 4%, 5%, 6%, 7%, 8%, 9%, 10%, 11%, 12%, 13%, 14%, 15%, 20%, etc.

In another embodiment, the cream may include a beneficial substance, such as ibuprofen, and one or more of the following: water (20-80%), L-arginine hydrochloride(0-25%), sodium chloride (0-25%), potassium chloride (0-25%), glyeryl steareate (0-15%), cetyl alcohol (0-15%), squalene (0-15%), isopropyl mysterate (0-15%), oleic acid (0-15%), Tween 20 (0-10 %), and/or butanediol (0-10%). The percentages of each compound can vary (or the compound may be absent in some cases), for example, 1%, 2%, 3%, 4%, 5%, 6%, 7%, 8%, 9%, 10%, 11%, 12%, 13%, 14%, 15%, 20%, etc.

In some embodiments, the cream may include a beneficial substance, and one or more ionic salts at a concentration at least sufficient to produce a hostile biophysical environment with respect to the beneficial substance. For example, the cream may include one or more of (w/v): a charged and/or hydrogen bonding beneficial substance with systemic toxicity (0.001-30%), choline chloride (1-30%), sodium chloride (2-30%), and/or magnesium chloride (1-20% w/v). In another example, the cream may include one or more of (w/v): L-arginine hydrochloride (2.5-25%), choline chloride (10-30%), sodium chloride (5-20%), and/or magnesium chloride (5-20%). In still another example, the cream may include one or more of (w/v): creatine (0.001-30%), inosine (0.001-30%), choline chloride (1-30%), sodium chloride (2-30%), magnesium chloride (1-20%), L-arginine (0.1-25%), and/or theophylline (0.1-20%). In some cases, the cream may also contain L-arginine hydrochloride (0-12.5% w/v) and/or theophylline (0-10% w/v). The percentages of each compound can vary (or the compound may be absent in some cases), for example, 1%, 2%, 3%, 4%, 5%, 6%, 7%, 8%, 9%, 10%, 11%, 12%, 13%, 14%, 15%, 20%, etc. In these examples, choline chloride, sodium chloride and magnesium chloride can provide a high ionic strength environment.

In certain aspects of the invention, multiple treatments of the delivery vehicle may increase the duration of the effects of the beneficial substance, for example two, three, four, five, or more treatments may be applied, depending on the particular application. For example, with repeated administrations, the beneficial effects of each treatment may be extended up to ten or twenty hours after treatment, or more in some cases. Such treatments may be given at any suitable frequency, depending on the particular application, for example, every 4 hours, every 8 hours, every 12 hours, every 18 hours, every 1 day, every 2 days, every 3 days, every week, etc. For instance, the treatment may be provided between about 2 and about 30 times within a time period of about 30 days. In some cases, the first treatment may be given at a higher level or concentration than subsequent treatments.

The following examples are intended to illustrate certain embodiments of the present invention, but do not exemplify the full scope of the invention.

### EXAMPLE 1

In this example, a 57 year old woman with severe arthritis in her hands and fingers applied a cream comprising a hostile biophysical environment, along with 10% w/v ibuprofen and 12.5% w/v L-arginine, to her hands. She rubbed the cream into the skin of her hands and fingers until completely absorbed. Within 10 minutes she noticed substantial relief from the pain. Within 30 minutes the pain was completely gone. Pain relief persisted for several hours.

### EXAMPLE 2

In this example, a 37 year old man with shoulder pain applied a cream comprising a hostile biophysical environment, along with 10% w/v ibuprofen and 12.5% w/v L-arginine, to the painful shoulder. He rubbed the cream in until it was completely absorbed. Within 30 minutes the pain was completely gone. The pain never returned.

### EXAMPLE 3

A 54 year old woman with a severe headache in her right temple applied a cream comprising a hostile biophysical environment, 10% w/v ibuprofen, and 12.5% w/v L-arginine to the painful temple. She rubbed the cream in until completely absorbed. Within 10 minutes substantial relief of the headache was achieved. Within 20 minutes the pain was gone. The pain never returned.

### EXAMPLE 4

A 33 year old woman with a history of genital herpes infection was treated with a topical transdermal preparation of acyclovir. Herpes is characterized by outbreaks which start as a red, sometimes itching area and progress to open sores. The acyclovir preparation included a hostile biophysical environment, 2.5% w/v acyclovir, and 12.5 % w/v L-Arginine. This preparation was applied as soon as the red and sometimes itching areas appeared. This treatment resulted in regression of the insipient herpes outbreak, returning the area to normal within two days and preventing the open sores from developing.

### EXAMPLE 5

A 58 year old man suffering from claudication of the lower leg applied a cream comprising L-arginine hydrochloride (12.5% w/v), choline chloride (10% w/v), magnesium chloride (5% w/v), and sodium chloride (5% w/v) in a penetrating base to his legs nightly. After using it daily for three days, the cramps from claudication never recurred as long as he continued daily use of the cream.

### EXAMPLE 6

A 72 year old man with a twelve year history of PAD and claudication severely incapacitating him was treated in this example. He began daily use of a cream containing L-arginine hydrochloride (12.5% w/v), choline chloride (10% w/v), magnesium chloride (5% w/v), and sodium chloride (5% w/v) in a penetrating base to his lower legs. After three days of use the frequency of the attacks were markedly reduced, and after ten days the attacks had ceased. Continued daily use of the cream continued to prevent attacks.

### EXAMPLE 7

Circulatory impairment and its sequlae have long been known to be a major complication of diabetes. For instance, it has been shown that, in diabetes, the functionality of the endothelial nitric oxide (NO)/nitric oxide synthase (eNOS) system is impaired. NO is generated in the endothelium through the oxidation of the amino acid, L-arginine by the enzyme eNOS. NO causes vascular smooth muscle to relax resulting in increased blood flow. In addition to being a substrate of eNOS, L-Arginine facilitates the dimerization of two identical subunits of eNOS, forming a homodimer. The enzyme is only active in the dimeric form. Under proper conditions, dimerization occurs rapidly, on a timescale of minutes. Once formed the dimer is generally stable.

Subjects with diabetes may have abnormally low levels of L-Arginine and elevated levels of the eNOS inhibitor, asymmetric dimethylarginine (ADME) in their plasma. Though the value of increasing L-Arginine levels in cases of impaired circulation is now recognized, practical schemes for therapeutic use of L-Arginine have been illusive. In this example, it was determined whether supplying L-Arginine transdermally would improve vascular function of the feet in patients with diabetes, as indicated by flow and temperature.

The example was designed as a double-blind vehicle-controlled two-period crossover protocol, with washout periods of one week. Sixteen subjects were enrolled and thirteen completed the study (age 56 +/- 8 yr). After analyzing the data it was shown that the effect of L-arginine persisted throughout the washout periods (Tables 1 and 2, AU standing for Arbitrary Units). Because of this, except for the initial exposure of L-arginine on virgin feet, the analysis was altered to determine the effect from cumulative exposure to L-arginine throughout the protocol. Blood flow was measured at the metatarsal and Achilles area using a Doppler flow meter, and temperature was measured at the metatarsal and big toe areas using an infrared thermometer. The active cream was a water-based moisturizing vehicle containing 12.5% L-arginine hydrochloride in a hostile biophysical environment comprising a high concentrations of choline chloride, sodium chloride and magnesium chloride. The control vehicle was identical, except that the L-arginine was omitted.

At the first visit, after baseline measurements were made each subject rubbed active cream (4 mg of L-arginine/cm²) into one foot and vehicle into the other. After thirty minutes measurements were made again. A one week wash out period followed. Patients returned after the wash out period and flow and temperature measurements were made. They were then randomly given either active or placebo cream and told to rub it into their feet in the morning and evening every day for two weeks. At the end of two weeks subjects returned and again measurements were made. A second one week wash out period followed that third visit. At the end of that period subjects returned and measurements were made. They were given the cross over product and told again to rub it into their feet morning and evening for two weeks. The subjects returned for final flow and temperature measurements at the end of that period.

At the first visit flow was increased at the Achilles in the foot with active cream from 8.1 +/- 3.3 to 11.5+/- 5.5 (p=0.05) thirty minutes after application. In the foot that received placebo cream flow failed to increase (8.1 +/-1.4 vs. 8.3 +/- 2.2). Further, at the last visit the temperature at the metatarsal area had risen from the initial value of 82.0 +/-2.3 to 86.9 +/-2.4 (p<0.0001) and the temperature of the big toe had risen from the initial visit value of 74.4 +/- 4.2 to 82.4 +/-4.8 (p< 0.0001). At the last visit the flow at the metatarsal area had risen from 8.7 +/-4.3 to 11.6 +/- 5.5 (p<0.0001) and flow at the Achilles area had risen from 8.4 +/- 2.5 to 11.4 +/- 5.5 (p=0.02). While the failure of the L-arginine effect to wash out removed the opportunity for placebo control, the improvement in temperature and flow were substantial and highly statistically significant. Though puzzling, one explanation of the persistence of the L-arginine effect is that the local tissue concentration of L-arginine becomes high enough to cause inactive monomers of eNOS to form active dimers.

Thus, in the patients studied in this example with diabetes, treatment of their feet with a transdermal preparation of L-arginine improved both flow and temperature, and this effect was surprisingly long lasting. Such improvement of compromised local blood flow would be beneficial and could reduce the complications of the disease.

**Table 1. Effect of Transdermal L-Arginine Cream on Temperature**

| | Metatarsal (°F) | p vs. Visit 1 | Big Toe (°F) | p vs Visit 1 |
|---|---|---|---|---|
| Visit 1 | 82.0 +/- 2.3 | | 74.4 +/- 4.2 | |
| Visit 2 | 84.1 +/- 3.4 | 0.004 | 77.7 +/- 5.3 | 0.01 |
| Visit 3 | 87.0 +/- 2.4 | <0.0001 | 83.6 +/- 4.9 | <0.0001 |
| Visit 4 | 86.1 +/- 2.4 | <0.0001 | 80.6 +/- 5.4 | <0.0001 |
| Visit 5 | 86.9 +/- 2.4 | <0.0001 | 82.4 +/- 4.8 | <0.0001 |

**Table 2. Effect of Transdermal L-Arginine Cream on Flow**

| | Metatarsal (AU) | p vs. Visit 1 | Achilles (AU) | p vs. Visit 1 |
|---|---|---|---|---|
| Visit 1 | 8.7 +/- 4.3 | | 8.4 +/- 2.5 | |
| Visit 2 | 10.8 +/- 5.9 | NS | 8.5 +/- 3.9 | NS |
| Visit 3 | 10.8 +/- 4.8 | 0.05 | 9.2 +/- 3.9 | NS |
| Visit 4 | 11.6 +/- 8.3 | NS | 10.0 +/- 4.2 | 0.06 |
| Visit 5 | 11.6 +/- 5.5 | <0.0001 | 11 | |

### EXAMPLE 8

This example illustrates one method of preparing a transdermal formula of the invention including ibuprofen. The final composition is shown in Table 3. Of course, those of ordinary skill in the art will understand that percentages other than the ones listed below are also possible, according to other embodiments of the invention.

**Table 3. Example of a Transdermal Preparation**

| | |
|---|---|
| Water | 49% |
| L-Arginine Hydrochloride | 7.5% |
| Ibuprofen (sodium salt) | 7.5% |
| Sodium Chloride | 10% |
| Potassium Chloride | 5% |
| Glyeryl Steareate (SE) | 7% |
| Cetyl Alcohol | 7% |
| Squalene | 2% |
| Isopropyl Mysterate | 1% |
| Oleic Acid | 1% |
| Tween 20 | 2% |
| Butanediol | 1% |

To prepare the formulation in this example, sodium chloride, potassium chloride, L-arginine and ibuprofen were mixed in water, then heated to 74 degrees C with rapid mixing. In a separate container, the remaining ingredients were mixed together and heated to 74 degrees C. The other ingredients were then added to the water phase at 74 degrees C with rapid mixing. The mixture was then cooled to room temperature with continued mixing. At this point, an emulsion formed with a relatively thin consistency. The emulsion was then homogenized at high speed at room temperature to thicken the consistency.

According to aspects of the invention described and illustrated herein, beneficial substance(s) (e.g., pharmaceutical agent(s)) may be provided (e.g., in a delivery vehicle) at a concentration of between about 0.1% and about 25% (for example at a concentration of 0.1%, 0.5%, 1%, 2%, 3%, 4%, 5%, 6%, 7%, 8%, 9%, 10%, 11%, 12%, 13%, 14%, 15%, 20%, etc.). However, higher (e.g., above 25%, 30%, 40%, 50% or higher) or lower (e.g., below 0.1%, 0.05% or lower) concentrations of beneficial substance(s) may be used. As used herein (for a beneficial substance or any other compound described herein) a concentration % may be a % by weight, a % by volume, or a % weight by volume. As used herein, a beneficial substance may be, for example, a charged beneficial substance, a non-charged beneficial substance, a beneficial substance that forms hydrogen bonds, a beneficial substance that does not form hydrogen bonds, etc.

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and

equivalents thereto, the invention may be practiced otherwise than as specifically described and claimed. The present invention is directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present invention.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

Aspects (A) of the invention are listed below.
A1. A method, comprising an act of: applying, to a portion of the skin of a subject, a delivery vehicle comprising a pharmaceutical agent in a hostile biophysical environment.
2. The method of A1, wherein the hostile biophysical environment has an ionic strength of at least about 1 M.
3. The method of A1, wherein the hostile biophysical environment comprises a urea.
4. The method of A1, wherein the hostile biophysical environment comprises a carbohydrate.
5. The method of A1, wherein the hostile biophysical environment has a pH of at least about 9.
6. The method of A1, wherein the hostile biophysical environment has a pH of less than about 5.
7. The method of A1, wherein the hostile biophysical environment comprises a component having an octanol-water partition coefficient of at least about 1000.
8. The method of A1, wherein the hostile biophysical environment comprises a component having an octanol-water partition coefficient of less than about 10⁻³.
9. The method of A1, wherein the act of applying occurs for a duration sufficient to transport an effective dosage of the pharmaceutical agent to treat a medical condition.
10. The method of A1, wherein the pharmaceutical agent is one or more of naproxen, celecoxib, refecoxib, morphine, propoxyphene, oxycodone, hydrocodon, yohimbie, alprostadil, sildenafil, cialis, uprima, vardenaifl, dihydroergotamine, ergotamine, surnatripan, rizatriptan, zolmitriptan, finasteride, eflornithine, minoxidil, niacin, lidocaine, or benzocaine.
11. The method of A1, wherein the hostile biophysical environment is capable of driving the pharmaceutical agent through stratum corneum.
12. The method of A1, wherein the hostile biophysical environment has a pH between about 3 and about 11.
13. The method of A1, wherein the hostile biophysical environment comprises an ionic salt.
14. The method of A1, wherein the hostile biophysical environment comprises one or more of sodium chloride, choline chloride, magnesium chloride, calcium chloride.
15. The method of A1, wherein the hostile biophysical environment has an ionic strength of between about 0.25 M and about 15 M.
16. The method of 9, wherein the medical condition is one or more of sexual dysfunction, baldness, joint pain, muscle pain, migraine, ulcers, or arthritis.
17. The method of 16, wherein the medical condition is sexual dysfunction.
18. The method of 17, wherein the medical condition is erectile dysfunction.
19. The method of 17, wherein the medical condition is female sexual dysfunction.
20. The method of 17, wherein the pharmaceutical agent is one or more of yohimbie, alprostadil, sildenafil, cialis, uprima, or vardenaifl.
21. The method of 17, wherein the delivery vehicle is contained within a condom.
22. The method of 17, wherein the pharmaceutical agent is applied to the clitoris and/or the vagina.
23. The method of A1, wherein the hostile biophysical environment comprises a silicon-containing substance.
24. The method of A1, wherein the silicon-containing substance comprises one or more of polydimethylsiloxane, cyclopentasiloxane, dimethicol, or dimethicon.
25. The method of 9, wherein the medical condition is hair loss, and the pharmaceutical agent is one or more of finasteride, eflornithine, or minoxidil.
26. The method of 9, wherein the medical condition is selected from the group consisting of cramps, pain, and arthritis.
27. The method of 26, wherein the medical condition is joint pain.
28. The method of 26, wherein the medical condition is muscle pain.
29. The method of 26, wherein the medical condition is arthritis.
30. The method of 26, wherein the pharmaceutical agent is one or more of ibuprofen, naproxen, celecoxib, refecoxib, morphine, propoxyphene, oxycodone, or hydrocodon.
31. The method of 26, wherein the medical condition is pain, and wherein the pharmaceutical agent comprises an NSAID.
32. The method of 31, wherein the NSAID comprises ibuprofen.
33. The method of 26, wherein the medical condition is migraine, and wherein the pharmaceutical agent is one or more of dihydroergotamine, ergotamine, sumatripan, rizatriptan, or zolmitriptan.
34. The method of 9, wherein the medical condition is cancer.
35. The method of 34, wherein the cancer is selected from the group consisting of skin cancer, breast cancer, penile cancer, or testicular cancer.
36. The method of 34, wherein the pharmaceutical agent comprises a chemotherapeutic agent.
37. The method of 9, wherein the medical condition is a viral infection.
38. The method of 37, wherein the viral infection is herpes.
39. The method of 37, wherein the pharmaceutical agent comprises an antiviral agent.
40. The method of 39, wherein the antiviral agent comprises acyclovir.
41. The method of A1, wherein the pharmaceutical agent comprises a muscle improving agent.
42. The method of 41, wherein the muscle improving agent is present at a concentration of between about 0.1% and about 20% by weight/volume.
43. The method of 41, wherein the muscle improving agent comprises creatine.
44. The method of 41, wherein the muscle improving agent comprises inosine.
45. The method of 41, wherein the muscle improving agent comprises adenosine.
46. The method of 41, wherein the muscle improving agent comprises ribose.
47. The method of 41, wherein the muscle improving agent comprises phosphate.
48. The method of 41, wherein the muscle improving agent comprises adenine.
49. The method of 41, wherein the muscle improving agent comprises hypoxanthine.
50. The method of A1, wherein the pharmaceutical agent comprises an anabolic steroid.
51. The method of 50, wherein the anabolic steroid comprises one or more of testosterone, androstene, DHEA, androstenediol, or androstenedione.
52. The method of A1, wherein the pharmaceutical agent comprises ephedra.
53. The method of A1, wherein the pharmaceutical agent comprises ephedrine.
54. The method of A1, wherein the pharmaceutical agent comprises pseudoephedrine.
55. The method of A1, wherein the delivery vehicle further comprises an adjunct.
56. The method of 55, wherein the adjunct comprises theophylline.
57. The method of A1, wherein the delivery vehicle further comprises a nitric oxide donor.
58. The method of 57, wherein the nitric oxide donor comprises L-arginine.
59. The method of 57, wherein the nitric oxide donor is present in an amount effective to increase blood flow within the skin.
60. The method of A1, wherein the delivery vehicle is a cream.
61. The method of A1, wherein the delivery vehicle is a gel.
62. The method of A1, wherein the delivery vehicle is a lotion.
63. The method of A1, wherein the delivery vehicle further comprises one or more of water, mineral oil, glyceryl stereate, squalene, propylene glycol stearate, wheat germ oil, glyceryl stearate, isopropyl myristate, steryl stearate, polysorbate 60, propylene glycol, oleic acid, tocopherol acetate, collagen, sorbitan stearate, vitamin A, vitamin D, triethanolamine, methylparaben, aloe vera extract, imidazolidinyl urea, propylparaben, PND, or BHA.
A64. A delivery vehicle, comprising: a nitric oxide donor; and a pharmaceutical agent at a dosage effective to treat a localized medical condition, wherein the dosage is lower than the effective dosage of the pharmaceutical agent when taken orally.
65. The delivery vehicle of A64, wherein the pharmaceutical agent is one or more of naproxen, celecoxib, refecoxib, morphine, propoxyphene, oxycodone, hydrocodon, yohimbie, alprostadil, sildenafil, cialis, uprima, vardenaifl, dihydroergotamine, ergotamine, surnatripan, rizatriptan, zolmitriptan, finasteride, eflornithine, minoxidil, niacin, lidocaine, or benzocaine.
66. The delivery vehicle of A64, wherein the nitric oxide donor comprises L-arginine.
67. The delivery vehicle of 66, wherein the L-arginine is present at a concentration of at least 0.5% by weight/volume.
68. The delivery vehicle of 66, wherein the L-arginine is present at a concentration of between about 0.5% and about 25% by weight/volume.
69. The delivery vehicle of A64, wherein the delivery vehicle further comprises a hostile biophysical environment containing the pharmaceutical agent.
70. The delivery vehicle of 69, wherein the hostile biophysical environment has a pH between about 3 and about 11.
71. The delivery vehicle of 69, wherein the hostile biophysical environment comprises an ionic salt.
72. The delivery vehicle of 71, wherein the ionic salt is present in a concentration of between about 5% and about 50% by weight/volume.
73. The delivery vehicle of 69, wherein the hostile biophysical environment comprises one or more of sodium chloride, choline chloride, magnesium chloride, calcium chloride.
74. The delivery vehicle of 69, wherein the hostile biophysical environment has an ionic strength of between about 0.25 M and about 15 M.
75. The delivery vehicle of 69, wherein the hostile biophysical environment comprises a silicon-containing substance.
76. The delivery vehicle of 69, wherein the silicon-containing substance comprises one or more of polydimethylsiloxane, cyclopentasiloxane, dimethicol, or dimethicon.
77. The delivery vehicle of A64, wherein the medical condition is selected from the group consisting of cramps, pain, migraine, arthritis, swelling, sexual dysfunction, hair loss, skin ulcers, and migraine.
78. The delivery vehicle of A64, wherein the medical condition is sexual dysfunction and the pharmaceutical agent is one or more of yohimbie, alprostadil, sildenafil, cialis, uprima, or vardenaifl.
79. The delivery vehicle of 78, wherein the delivery vehicle is contained within a condom.
80. The delivery vehicle of 78, wherein the delivery vehicle further comprises a lubricant.
81. The delivery vehicle of A64, wherein the pharmaceutical agent is able to treat headache.
82. The delivery vehicle of A64, wherein the pharmaceutical agent comprises an NSAID.
83. The delivery vehicle of 82, wherein the NSAID comprises ibuprofen.
84. The delivery vehicle of A64, wherein the medical condition is hair loss, and the pharmaceutical agent is one or more of finasteride, eflornithine, or minoxidil.
85. The delivery vehicle of A64, further comprising a hair condition formula.
86. The delivery vehicle of A64, wherein the medical condition is selected from the group consisting of cramps, pain, and arthritis, and wherein the pharmaceutical agent is one or more of ibuprofen, naproxen, celecoxib, refecoxib, morphine, propoxyphene, oxycodone, or hydrocodon.
87. The delivery vehicle of A64, wherein the pharmaceutical agent comprises a chemotherapeutic agent.
88. The delivery vehicle of A64, wherein the pharmaceutical agent comprises an antiviral agent.
89. The delivery vehicle of A64, wherein the pharmaceutical agent comprises a muscle improving agent.
90. The delivery vehicle of 89, wherein the muscle improving agent comprises creatine.
91. The delivery vehicle of 89, wherein the muscle improving agent comprises inosine.
92. The delivery vehicle of 89, wherein the muscle improving agent comprises adenosine.
93. The delivery vehicle of 89, wherein the muscle improving agent comprises ribose.
94. The delivery vehicle of 89, wherein the muscle improving agent comprises phosphate.
95. The delivery vehicle of 89, wherein the muscle improving agent comprises adenine.
96. The delivery vehicle of 89, wherein the muscle improving agent comprises hypoxanthine.
97. The delivery vehicle of A64, wherein the pharmaceutical agent comprises an anabolic steroid.
98. The delivery vehicle of 97, wherein the anabolic steroid comprises one or more of testosterone, androstene, DHEA, androstenediol, or androstenedione.
99. The delivery vehicle of A64, wherein the pharmaceutical agent comprises ephedra.
100. The delivery vehicle of A64, wherein the pharmaceutical agent comprises ephedrine.
101. The delivery vehicle of A64, wherein the pharmaceutical agent comprises pseudoephedrine.
102. The delivery vehicle of A64, further comprising an adjunct.
103. The delivery vehicle of 102, wherein the adjunct comprises theophylline.
104. The delivery vehicle of A64, wherein the delivery vehicle is a cream.
105. The delivery vehicle of A64, wherein the delivery vehicle is a gel.
106. The delivery vehicle of A64, wherein the delivery vehicle is a lotion.
107. The delivery vehicle of A64, further comprising one or more of water, mineral oil, glyceryl stereate, squalene, propylene glycol stearate, wheat germ oil, glyceryl stearate, isopropyl myristate, steryl stearate, polysorbate 60, propylene glycol, oleic acid, tocopherol acetate, collagen, sorbitan stearate, vitamin A, vitamin D, triethanolamine, methylparaben, aloe vera extract, imidazolidinyl urea, propylparaben, PND, or BHA.
A108. A delivery vehicle, comprising: a nitric oxide donor; and a pharmaceutical agent able to treat one or more medical conditions selected from the group consisting of cramps, pain, migraine, arthritis, swelling, sexual dysfunction, hair loss, skin ulcers, and migraine.
109. The delivery vehicle of A108, wherein the nitric oxide donor comprises L-arginine.
110. The delivery vehicle of A108, wherein the medical condition is selected from the group consisting of cramps, pain, and arthritis, and wherein the pharmaceutical agent is one or more of ibuprofen, naproxen, celecoxib, refecoxib, morphine, propoxyphene, oxycodone, or hydrocodon.
111. The delivery vehicle of A108, wherein the medical condition is sexual dysfunction and the pharmaceutical agent is one or more of yohimbie, alprostadil, sildenafil, cialis, uprima, or vardenaifl.
112. The delivery vehicle of A108, wherein the delivery vehicle is contained within a condom.
113. The delivery vehicle of A108, wherein the delivery vehicle further comprises a lubricant.
114. The delivery vehicle of A108, wherein the medical condition is hair loss, and the pharmaceutical agent is one or more of finasteride, eflornithine, or minoxidil.
115. The delivery vehicle of A108, further comprising one or more of water, mineral oil, glyceryl stereate, squalene, propylene glycol stearate, wheat germ oil, glyceryl stearate, isopropyl myristate, steryl stearate, polysorbate 60, propylene glycol, oleic acid, tocopherol acetate, collagen, sorbitan stearate, vitamin A, vitamin D, triethanolamine, methylparaben, aloe vera extract, imidazolidinyl urea, propylparaben, PND, or BHA.

## Claims

1. A delivery vehicle comprising a pharmaceutical agent in an environment of ionic strength of at least about 1 M, for use in a method of treating sexual dysfunction, the method comprising applying the delivery vehicle to a portion of the skin of a subject, wherein the pharmaceutical agent is yohimbine, alprostadil, sildenafil, cialis, uprima or vardenafil.

2. The delivery vehicle of claim 1, wherein the environment is capable of driving the pharmaceutical agent through stratum corneum.

3. The delivery vehicle of claim 1 or 2, wherein the sexual dysfunction is erectile dysfunction or is female sexual dysfunction.

4. The delivery vehicle of any one of claims 1-3, wherein the method comprises applying the delivery vehicle to the clitoris and/or the vagina.

5. The delivery vehicle of any one of claims 1-4, wherein the environment comprises one or more of sodium chloride, choline chloride, magnesium chloride, lithium chloride, and calcium chloride.

6. The delivery vehicle of any one of claims 1-5, wherein the environment comprises sodium chloride.

7. The delivery vehicle of any one of claims 1-6, wherein the environment further comprises a nitric oxide donor.

8. The delivery vehicle of claim 7, wherein the nitric oxide donor comprises L-arginine hydrochloride.

9. The delivery vehicle of claim 7, wherein the nitric oxide donor comprises an L-arginine salt.

10. The delivery vehicle of any one of claims 1-9, wherein the environment comprises citric acid.

11. The delivery vehicle of any one of claims 7-9, wherein the environment further comprises one or more of polydimethylsiloxane, cyclopentasiloxane, dimethicol, and dimethicon.

12. The delivery vehicle of any one of claims 1-11, wherein the delivery vehicle is a cream.

13. The delivery vehicle of any one of claims 1-11, wherein the delivery vehicle is a gel or a lotion.

14. The delivery vehicle of any one of claims 1-13, wherein the pharmaceutical agent is sildenafil.
